(19) Europäisches Patentamt
European Patent Office
Office européen des brevets

(11) **EP 2 149 326 A1**

(12) **EUROPEAN PATENT APPLICATION**
published in accordance with Art. 153(4) EPC

(43) Date of publication:
**03.02.2010 Bulletin 2010/05**

(51) Int Cl.:
**A61B 1/00** (2006.01)    **A61B 1/04** (2006.01)
**G06T 1/00** (2006.01)

(21) Application number: **08740251.7**

(22) Date of filing: **11.04.2008**

(86) International application number:
**PCT/JP2008/057154**

(87) International publication number:
**WO 2008/139803 (20.11.2008 Gazette 2008/47)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **08.05.2007 JP 2007123826**

(71) Applicant: **Olympus Corporation**
**Tokyo 151-0072 (JP)**

(72) Inventor: **KITAMURA, Makoto**
**Tokyo 151-0072 (JP)**

(74) Representative: **Schmidt, Steffen**
**Wuesthoff & Wuesthoff**
**Patentanwälte**
**Schweigerstrasse 2**
**81541 München (DE)**

(54) **IMAGE PROCESSING DEVICE AND IMAGE PROCESSING PROGRAM**

(57)    An image processing apparatus (1) according to an embodiment processes a series of observation images on which an esophagus, a stomach, a small intestine, and a large intestine are sequentially captured as a plurality of observation targets. The image processing apparatus includes an organ identifying unit (4a) that identifies that, at least on the basis of compressed information based on compressed image data of an image to be processed among the series of observation images, the organ captured on an image to be processed corresponds to which organ of the esophagus or the stomach, the small intestine, and the large intestine. The organ identifying unit (4a) sequentially selects an image to be processed among the series of observation images, and sequentially identifies an organ captured on the image to be processed.

## FIG.1

EP 2 149 326 A1

**Description**

TECHNICAL FIELD

The present invention relates to an image processing apparatus and an image processing program. Particularly, the present invention relates to an image processing apparatus and an image processing program for processing a series of observation images on which a plurality of observation targets are sequentially captured.

BACKGROUND ART

A capsule endoscope that observes the inside of a subject has been recently developed as one of image capturing devices that can sequentially capture a plurality of observation images. During the period after the capsule endoscope is swallowed into the subject and until the capsule endoscope is naturally discharged from the subject, the capsule endoscope includes an imaging function and captures the inside images of an esophagus, a stomach, a small intestine, a large intestine, and so on, while sequentially moving the inside organs in accordance with a peristalsis or the like. A doctor, a nurse, or the like can cause a display to display the captured images as observation images and can observe the inside of the subject based on the observation images.

The number of the series of observation images acquired by the capsule endoscope is enormous usually. A doctor, a nurse, or the like requires a lot of time and an effort to observe the series of observation images. On the other hand, there has been developed an image displaying apparatus that can display only observation images on which desired observation regions are captured and thus can effectively observe a series of observation images (for example, see Patent Document 1). The image displaying apparatus detects a villus, excrement, or the like captured on the observation images by using a frequency analysis, a texture analysis, or the like. Because the series of observation images are classified into stomach images, small intestine images, large intestine images, and so on based on the detection results, only observation images on which a desired organ is captured can be displayed.

Patent Document 1: Japanese Patent Application Laid-open No. 2006-320585

DISCLOSURE OF INVENTION

PROBLEM TO BE SOLVED BY THE INVENTION

However, because a frequency analysis and a texture analysis generally require a lot of processing time, the image displaying apparatus disclosed in Patent Document 1 cannot quickly display the series of observation images after acquiring the observation images. For this reason, there has been a problem that an observer must spend a lot of waiting time to observe a desired observation image.

The present invention has been made in view of the above problems, and an object of the invention is to provide an image processing apparatus and an image processing program that can quickly identify the observation target in a series of observation images on which a plurality of observation targets are sequentially captured.

MEANS FOR SOLVING PROBLEM

To achieve the above object, an image processing apparatus according to the present invention processes a series of observation images on which a plurality of observation targets are sequentially captured. The image processing apparatus includes a target identifying unit that identifies, at least on the basis of compressed information based on compressed image data of an image to be processed among the series of observation images, an observation target captured on the image to be processed.

An image processing program according to the present invention causes an image processing apparatus that processes a series of observation images on which a plurality of observation targets are sequentially captured, to process the series of observation images. The image processing program causes the image processing apparatus to perform: identifying, at least on the basis of compressed information based on compressed image data of an image to be processed among the series of observation images, an observation target captured on the image to be processed.

EFFECT OF THE INVENTION

According to the image processing apparatus and the image processing program according to the present invention, the observation target in a series of observation images on which a plurality of observation targets are sequentially captured can be identified by a simple process.

BRIEF DESCRIPTION OF DRAWINGS

FIG. 1 is a block diagram illustrating the configuration of an image processing apparatus according to a first embodiment.

FIG. 2 is a flowchart illustrating an image processing procedure performed by the image processing apparatus.

FIG. 3 is a flowchart illustrating an organ identification processing procedure according to the first embodiment.

FIG. 4-1 is a diagram illustrating file sizes of a series of observation images.

FIG. 4-2 is a diagram illustrating a moving average of the file sizes of the series of observation images.

FIG. 4-3 is a diagram illustrating a moving average of file-size change amounts of the series of observation images.

FIG. 5 is a flowchart illustrating an organ identification processing procedure according to a second embodiment.

FIG. 6 is a diagram illustrating DCT coefficients in an 8 x 8 pixel block.

FIG. 7 is a block diagram illustrating the configuration of an image processing apparatus according to a third embodiment.

FIG. 8 is a flowchart illustrating an organ identification processing procedure according to the third embodiment.

FIG. 9 is a diagram explaining reference data.

EXPLANATIONS OF LETTERS OR NUMERALS

| 1, 10 | Image processing apparatus |
| --- | --- |
| 2 | Input unit |
| 3, 13 | Storing unit |
| 3a | Observation image storing unit |
| 4, 14 | Image processing unit |
| 4a, 14a | Organ identifying unit |
| 5 | Output unit |
| 6, 16 | Control unit |
| 13b | Reference data storing unit |

BEST MODE(S) FOR CARRYING OUT THE INVENTION

Exemplary embodiments of an image processing apparatus and an image processing program according to the present invention will be explained below in detail with reference to the accompanying drawings. In the present embodiments, the image processing apparatus according to the present invention processes a series of observation images, on which the insides of an esophagus, a stomach, a small intestine, and a large intestine are sequentially captured, as a series of observation images on which a plurality of observation targets are sequentially captured. However, observation images, which can be processed by the image processing apparatus according to the present invention, are not limited to observation images on which such digestive organs are captured. The present invention is not limited to the embodiments explained below.

Moreover, in the drawings, the same reference numerals are given to the same components.

(First Embodiment)

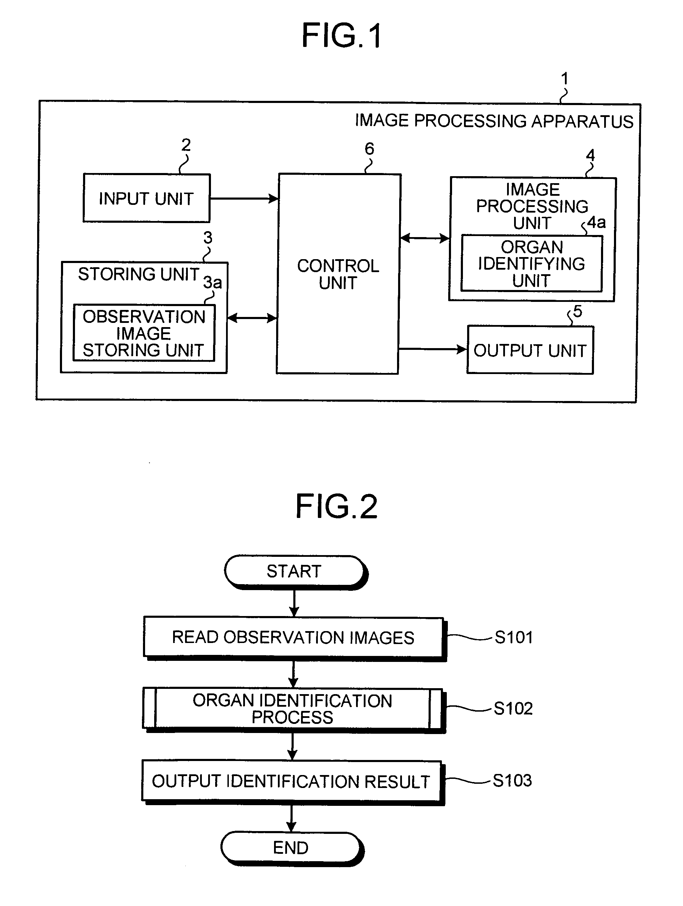

First, an image processing apparatus according to the first embodiment of the present invention will be explained. FIG. 1 is a block diagram illustrating the main configuration of an image processing apparatus 1 according to the first embodiment. As illustrated in FIG. 1, the image processing apparatus 1 includes an input unit 2, a storing unit 3, an output unit 5, an image processing unit 4, and a control unit 6. The input unit 2 receives various types of information including an image, the storing unit 3 stores therein the same, and the output unit 5 outputs the same. The image processing unit 4 processes an image stored in the storing unit 3. The control unit 6 is electrically connected to the units and controls a process and an operation of each unit.

The input unit 2 can be a data communication interface. The data communication interface inputs image data of a series of observation images to be processed into the control unit 6. In the first embodiment, the image data of the series of observation images are input into the control unit 6 as compressed image data. Moreover, the input unit 2 includes various types of input devices, and inputs various types of information such as a process parameter that is used in the control unit 6.

The storing unit 3 is configured using a hard disk, a ROM, a RAM, and so on. The storing unit 3 stores therein various types of information such as various types of processing programs executed by the control unit 6, various types of processing parameters for use in the control unit 6, and processing results of the control unit 6. Particularly, the storing unit 3 includes an observation image storing unit 3a that stores therein the series of observation images input via the input unit 2. Moreover, the storing unit 3 includes a portable storage medium attachable to and removable from the image processing apparatus 1 and can acquire image data without via the input unit 2 to store the series of observation images.

The image processing unit 4 is realized by, for example, a CPU. The image processing unit 4 performs various types of image processing on the series of observation images stored in the observation image storing unit 3a on the basis of a predetermined image processing program executed by the control unit 6. Particularly, the image processing unit 4 includes an organ identifying unit 4a that acts as a target identifying unit that identifies an observation target captured on each observation image. Specifically, the organ identifying unit 4a identifies that the observation target captured on each observation image corresponds to which organ of an esophagus or a stomach, a small intestine, and a large intestine.

The output unit 5 is configured using various types of displays such as a liquid crystal display. The output unit 5 informs a user of an identification result performed by the organ identifying unit 4a. Moreover, the output unit 5 includes a data communication interface. The data communication interface can output the identification result performed by the organ identifying unit 4a to an external device. The output unit 5 can further display the series of observation images and various types of information.

The control unit 6 is realized by a CPU. The control unit 6 controls a process and an operation of each unit included in the image processing apparatus 1 by executing a predetermined processing program stored in the storing unit 3. Particularly, by executing a predetermined image processing program stored in the storing unit 3, the control unit 6 causes the image processing unit 4 to process the series of observation images, the organ identifying unit 4a to identify the type of an organ captured on each observation image, and the output unit 5 to output the identification result.

Next, an image processing procedure performed by the image processing apparatus 1 will be explained. FIG. 2 is a flowchart of a processing procedure of processing the series of observation images stored in the observation image storing unit 3a in a situation where the control unit 6 executes the predetermined image processing program. As illustrated in FIG. 2, the image processing unit 4 firstly reads compressed image data of the series of observation images from the observation image storing unit 3a (Step S101), and the organ identifying unit 4a identifies the organ captured on each observation image (an organ identification process) (Step S102). After that, the control unit 6 causes the output unit 5 to output an identification result performed in the organ identification process (Step S103), and terminates a series of processes.

In the organ identification process of Step S102, the organ identifying unit 4a identifies whether an organ captured on each observation image is either an esophagus or a stomach, or either a small intestine or a large intestine on the basis of the file size of compressed image data obtained by compressing and encoding the observation image. The inside of an esophagus or a stomach does not have unevenness comparatively. Therefore, an observation image on which an esophagus or a stomach is captured has a characteristic that a correlation between each pixel and a peripheral pixel thereof is high, as compared to an observation image on which a small intestine or a large intestine is captured. The organ identifying unit 4a identifies the height of the correlation by using the file size of the compressed image data. According to the identification, the organ identifying unit 4a identifies whether an organ captured on each observation image is either an esophagus or a stomach, or either a small intestine or a large intestine.

In general, it has been known that the height of the correlation between each pixel and a peripheral pixel thereof is expressed by entropy. Entropy H(f) is calculated by the following Equation (1) by using a bit stream r of a peripheral pixel of a target pixel and a probability p(r;f) by which the target pixel has a pixel value f. The entropy H(f) expressed by Equation (1) is the entropy of a Markov information source.

$$H(f) = -\log_2(p(r;f)) \quad \cdots \quad (1)$$

The entropy H(f) corresponding to each pixel can be obtained by performing the operation of Equation (1) on the whole of the image. When the value of entropy H(f) is large as the whole tendency of image, it can be said that the image is a low correlation between each pixel and a peripheral pixel thereof. When the value of entropy H(f) is small, it can be said that the image has a high correlation between each pixel and a peripheral pixel thereof.

Moreover, the entropy of image has the following property. That is, when the value of entropy is large, an information amount of the image is large and a compression encoding rate is low. On the other hand, when the value of entropy is small, an information amount of the image is small and a compression encoding rate is high. In other words, when comparing the file sizes of compressed image data for two images of which the file sizes before compression encoding are equal, it can be said that the image, of which the file size of the compressed image data is large, has a relatively low compression encoding rate and has large entropy. On the other hand, it can be said that the image, of which the file size of the compressed image data is small, has a relatively high compression encoding rate and has small entropy.

As described above, it can be identified that the image of which the file size of the compressed image data is large has a low correlation, and the image of which the file size of the compressed image data is small has a high correlation. By utilizing the characteristic, the organ identifying unit 4a identifies whether an organ captured on an observation image is either an esophagus or a stomach, or either a small intestine or a large intestine, on the basis of the file size of the compressed image data of the observation image.

When usually computing the entropy of each pixel by using Equation (1), an enormous processing time is needed. On the contrary, in the first embodiment, because the series of observation images are previously compressed and encoded and then are stored, the file size of the compressed image data of the observation image can be easily obtained. Therefore, the organ identifying unit 4a can quickly identify the height of the correlation by a simple process and can identify an organ captured on the observation image, as compared to a situation where the entropy is computed and the correlation between each pixel and a peripheral pixel thereof is calculated.

Moreover, in the organ identification process of Step S102, the organ identifying unit 4a identifies whether an organ captured on an observation image is a small intestine or a large intestine on the basis of the change amount of the file size of the compressed image data of the observation image. The inside of the large intestine is filled with excrements. Therefore, when observation images are acquired by a capsule endoscope, for example, the movement of the capsule endoscope is slow, so that the file size between observation images consecutive in chronological order does not almost change. On the other hand, because the capsule endoscope can be smoothly moved in the small intestine as compared to the movement in the large intestine, the file size between observation images consecutive in chronological order changes remarkably. By utilizing the characteristic, the organ identifying unit 4a identifies whether an observation target captured on an observation image is a small intestine or a large intestine, on the basis of the size of the change amount of the file size between the observation images consecutive in chronological order.

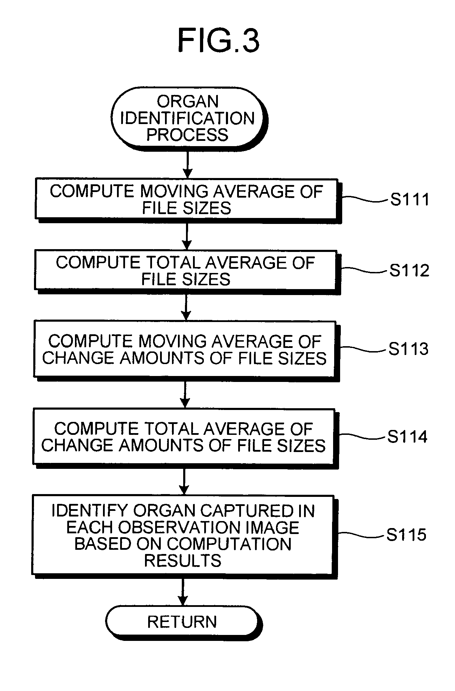

Next, the specific processing procedure of the organ identification process performed by the organ identifying unit 4a will be explained. FIG. 3 is a flowchart illustrating the organ identification processing procedure. As illustrated in FIG. 3, on the basis of the file sizes of compressed image data of a series of observation images, the organ identifying unit 4a first computes a moving average of the file sizes (Step S111) and computes a total average of the file sizes (Step S112). Furthermore, the organ identifying unit 4a computes change amounts of the file sizes between consecutive observation images in the series of observation images. The organ identifying unit 4a computes a moving average of the change amounts of the file sizes (Step S113) and computes a total average of the change amounts of the file sizes (Step S114). After that, on the basis of computation results performed at Steps S111 to S113, the organ identifying unit 4a identifies an organ captured on each observation image (Step S115) and terminates the organ identification process. Then, the organ identifying unit 4a returns the system control to Step S102.

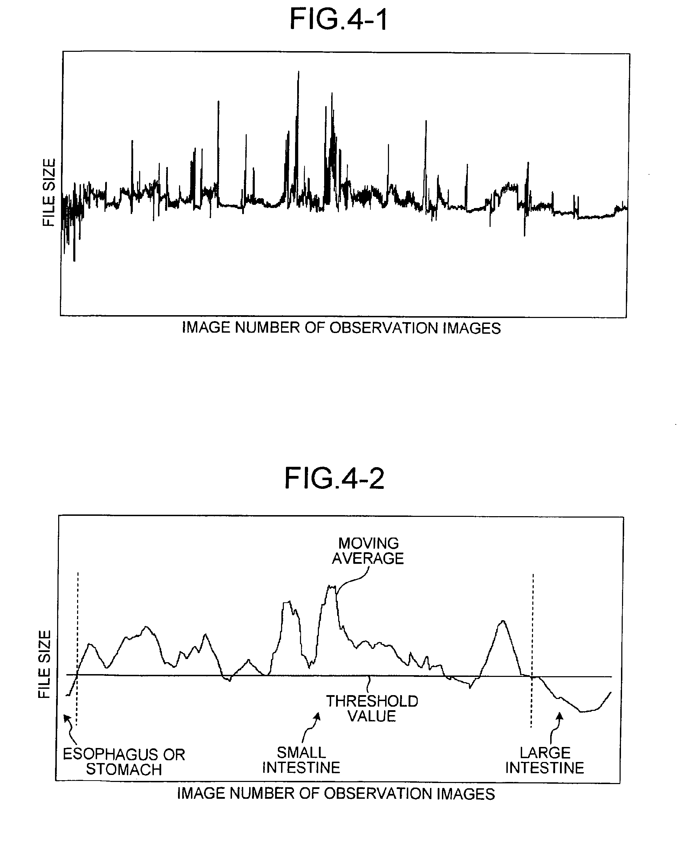

At Step S111, the organ identifying unit 4a computes, for an image to be processed among the series of observation images, a size average that is an average of file sizes of a plurality of observation images that includes the image to be processed and are adjacent to the image to be processed in time series. Then, the organ identifying unit 4a associates the computed size average with the image to be processed. In the first embodiment, the organ identifying unit 4a computes the size average by using, for example, 100 observation images adjacent to the image to be processed in time series among the series of observation images. However, the number of observation images for computing the size average can be set to a suitable number in accordance with an imaging interval for capturing the series of observation images. The organ identifying unit 4a sequentially selects an image to be processed among the series of observation images and computes a size average for each selected image to be processed. In this way, the organ identifying unit 4a obtains moving averages of file sizes over the series of observation images. Specifically, at Step S111, the organ identifying unit 4a obtains moving averages of file sizes as illustrated in FIG. 4-2, for example, on the basis of file size information of the series of observation images illustrated in FIG. 4-1.

At Step S113, the organ identifying unit 4a computes, for an image to be processed among the series of observation images, a change-amount average that is an average of change amounts of file sizes between the respective observation images of a plurality of observation images that includes the image to be processed and are adjacent to the image to be processed in time series. Then, the organ identifying unit 4a associates the computed change-amount average with the image to be processed. In the first embodiment, the organ identifying unit 4a computes the change-amount average by using, for example, 100 observation images adjacent to the image to be processed in time series among the series of observation images. However, the number of observation images for computing the change-amount average can be set to a suitable number in accordance with an imaging interval for capturing the series of observation images. The organ identifying unit 4a sequentially selects an image to be processed among the series of observation images and computes a change-amount average for each selected image to be processed. In this way, the organ identifying unit 4a obtains moving averages of change amounts of file sizes over the series of observation images.

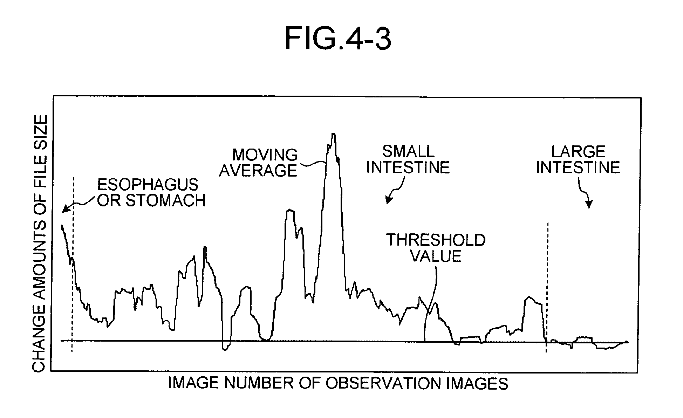

Specifically, at Step S113, the organ identifying unit 4a obtains moving averages of change amounts of file sizes as illustrated in FIG. 4-3, for example, on the basis of file size information of the series of observation images illustrated in FIG. 4-1.

At Step S115, the organ identifying unit 4a first identifies, for an image to be processed among the series of observation images, whether an organ captured on the image to be processed is either an esophagus or a stomach, or either a small intestine or a large intestine in accordance with a magnitude relation between the size average computed at Step S111 and a predetermined size criterion. Specifically, the organ identifying unit 4a computes a threshold value $T_{Fsize}$ that functions as a size criterion by using the following Equation (2) (see FIG. 4-2) on the basis of a total average $F_{sizeAve}$ computed at Step S112 and a variable M set previously and determines whether a size average $F_{size}$ satisfies the following Inequality (3) with respect to the threshold value $T_{Fsize}$.

$$T_{Fsize} = F_{sizeAve} + M \quad \cdots \quad (2)$$

$$F_{size} < T_{Fsize} \quad \cdots (3)$$

When Inequality (3) is satisfied, the organ identifying unit 4a identifies that an organ captured on the image to be processed is an esophagus or a stomach. When Inequality (3) is not satisfied, the organ identifying unit 4a identifies that the organ is a small intestine or a large intestine. Then, the organ identifying unit 4a associates the identification result with the image to be processed. Furthermore, the organ identifying unit 4a sequentially selects an image to be processed among the series of observation images and performs a similar identification process for each selected image to be processed. In this way, the organ identifying unit 4a identifies whether an organ captured on each observation image of the series of observation images is either an esophagus or a stomach, or either a small intestine or a large intestine.

In addition, when it is clear that the series of observation images have been captured in the order of stomach, small intestine, and large intestine, the organ identifying unit 4a sequentially selects an image to be processed from the leading image of the series of observation images. When an observation image not satisfying Inequality (3) is first found, the organ identifying unit 4a identifies that all observation images after the first found observation image are an image on which a small intestine or a large intestine is captured. In this way, the organ identifying unit 4a can more quickly identify whether an observation image is an image on which either an esophagus or a stomach is captured, or an image on which either a small intestine or a large intestine is captured. In other words, when the imaging sequence is predetermined for plural kinds of observation targets, the organ identifying unit 4a identifies that an observation target is a target having the former imaging sequence, and after the organ identifying unit 4a identifies that an observation target is the next observation target, the organ identifying unit 4a can identify that an observation target to be identified after that is the next observation target or an observation target having the next imaging sequence.

Next, the organ identifying unit 4a identifies whether an organ captured on an image to be processed is a small intestine or a large intestine for the image to be processed among the series of observation images that have been identified to be images of a small intestine or a large intestine, in accordance with a magnitude relation between the change-amount average computed at Step S113 and a predetermined change-amount criterion. Specifically, the organ identifying unit 4a computes a threshold value $T_{FsizeDiff}$ that functions as a change-amount criterion by using the following Equation (4) (see FIG. 4-3) on the basis of a total average $F_{sizeDiffAve}$ computed at Step S114 and a variable N set previously and determines whether a change-amount average $F_{sizeDiff}$ satisfies the following Inequality (5) for the threshold value $T_{FsizeDiff}$.

$$T_{FsizeDiff} = F_{sizeDiffAve} + N \quad \cdots \quad (4)$$

$$F_{sizeDiff} < T_{FsizeDiff} \quad \cdots \quad (5)$$

The organ identifying unit 4a identifies that an organ captured on the image to be processed is a large intestine when Inequality (5) is satisfied and identifies that the organ is a small intestine when Inequality (5) is not satisfied. Then, the organ identifying unit 4a associates the identification result with the image to be processed. Furthermore, the organ identifying unit 4a sequentially selects an image to be processed among the series of observation images that have been identified in first to be captured thereon either a small intestine or a large intestine, and performs a similar identification for the each selected image to be processed. In this way, the organ identifying unit 4a identifies whether an organ captured on each observation image is a small intestine or a large intestine. In this manner, the organ identifying unit 4a can identify that an organ captured on each image of the series of observation images corresponds to which organ of an esophagus or a stomach, a small intestine, and a large intestine, and associate an identification result with each observation image.

As indicated by Equation (2), the total average $F_{sizeAve}$ of file sizes is used to compute the threshold value $T_{Fsize}$ that functions as the size criterion. The reason is to reduce an influence induced by an individual difference because each subject has the individual difference with respect to a special feature of an organ. Similarly, as indicated by Equation (4), the total average $F_{sizeDiffAve}$ of change amounts of file sizes is used to compute the threshold value $T_{FsizeDiff}$ that functions as the change-amount criterion. The reason is to reduce an influence induced by the individual difference. Moreover, the variables M and N are set by being inputted by the observer through the input unit 2. Therefore, the variables can be changed appropriately.

As described above, the image processing apparatus 1 according to the first embodiment includes the organ identifying unit 4a that identifies an organ that is an observation target captured on an image to be processed on the basis of a file size that is compressed information based on compressed image data of the image to be processed among the series of observation images. The organ identifying unit 4a sequentially selects an image to be processed among the series of observation images and identifies the type of an organ captured on each selected image to be processed. Therefore, the organ identifying unit 4a can quickly identify an organ captured on each observation image of the series of observation images on which a plurality of internal organs such as an esophagus, a stomach, a small intestine, and a large intestine are sequentially captured. Moreover, the organ identifying unit 4a associates the identification result with each observation image. Therefore, the series of observation images can be identified for each captured organ.

In the organ identification process as described above, the organ identifying unit 4a identifies an organ captured on each observation image in block by using Step S115. However, the organ identifying unit 4a can individually perform the identification of Inequality (3) and the identification of Inequality (5). For example, the organ identifying unit 4a performs the identification of Inequality (3) just after Step S112. In this way, the organ identifying unit 4a can perform Step S113 on only the observation image that has been identified to be a image of a small intestine or a large intestine. Therefore, the organ identification process can be performed more quickly.

Moreover, in the organ identification process as described above, the organ identifying unit 4a sequentially performs the identification of Inequality (3) and the identification of Inequality (5) at Step S115. However, the organ identifying unit 4a can perform the identifications in block. For example, the organ identifying unit 4a can calculate a feature vector ($F_{size}$, $F_{sizeDiff}$) expressed by the size average $F_{size}$ and the change-amount average $F_{sizeDiff}$ for each image to be processed and identify the type of an organ in accordance with an area to which the feature vector belongs on a feature space. Specifically, the organ identifying unit 4a can identify that either an esophagus or a stomach is captured when the feature vector ($F_{size}$, $F_{sizeDiff}$) is in an area satisfying Inequality (3). The organ identifying unit 4a can identify that a large intestine is captured when the feature vector is in an area that is other than the area satisfying Inequality (3) and satisfies Inequality (5). The organ identifying unit 4a can identify that a small intestine is captured when the feature vector is in an area other than these areas.

Moreover, in the organ identification process as described above, the organ identifying unit 4a identifies an organ on the basis of a size average and a change-amount average of file sizes of the plurality of observation images. However, the averages are not necessarily used. The organ identifying unit 4a can identify an organ, for example, on the basis of an individual file size and a change amount of the individual file size. In this way, when comparatively loose identification accuracy is requested, an organ identification process can be performed more quickly.

(Second Embodiment)

Next, the image processing apparatus according to the second embodiment of the present invention will be explained. In the first embodiment as described above, the organ identifying unit 4a identifies an organ captured on each observation image on the basis of file sizes of compressed image data of observation images and change amounts thereof. In the second embodiment, the organ identifying unit 4a identifies an organ on the basis of DCT coefficients computed at the time of decompression of compressed image data and change amounts thereof. The image processing apparatus according to the second embodiment has the same configuration as that of the image processing apparatus 1, and the organ identifying unit 4a performs an organ identification process of Step S102 on the basis of a DCT coefficient instead of a file size.

An esophagus or a stomach usually has a mucous membrane surface that has a little unevenness and is flat as compared to a small intestine. On the other hand, the surface of the small intestine has much unevenness due to villus. For this reason, an observation image on which a stomach is captured dominantly has a low frequency component, and an observation image on which a small intestine is captured dominantly has a high frequency component. In the second embodiment, the organ identifying unit 4a identifies whether the organ captured on the observation image is either an esophagus or a stomach, or either a small intestine or a large intestine by using the property. Specifically, when the series of observation images are stored as compressed image data compressed by a DCT compression encoding mode such as JPEG, the organ identifying unit 4a performs an identification on the basis of a plurality of DCT coefficients obtained by an inverse DCT transform that is performed at the time of decompression of the compressed image data.

In addition, because a large intestine includes therein excrement, the movement of a capsule endoscope is stagnant when observation images are acquired by the capsule endoscope, for example. Therefore, a frequency component between observation images consecutive in chronological order does not almost change. On the other hand, because the capsule endoscope can smoothly move inside the small intestine as compared to the large intestine, a frequency component between observation images consecutive in chronological order has a remarkable change. By utilizing the characteristic, the organ identifying unit 4a identifies whether the organ captured on the observation image is a small intestine or a large intestine on the basis of the size of the change amount of the frequency component between the observation images consecutive in chronological order. Specifically, the organ identifying unit 4a performs an identification on the basis of the change amount of the DCT coefficient between the observation images consecutive in chronological order when the series of observation images are stored as compressed image data compressed by the DCT compression encoding mode.

Generally, a method of calculating a power spectrum by using Fourier transform is known well as a technique for obtaining frequency component information in an image. However, because Fourier transform has many computation processes, Fourier transform usually requires an enormous processing time. On the contrary, when a frequency component is identified by using a DCT coefficient as described above, the DCT coefficient can be computed at the time of the decompression process of the compressed image data without requiring special arithmetic processing to identify the frequency component. Moreover, a process of computing the DCT coefficient is simple. Therefore, the process can be performed in a short time. The process of computing the DCT coefficient can quickly identify a frequency component in an observation image and can identify the type of an organ captured on the observation image as compared to the case of using the power spectrum calculated by Fourier transform.

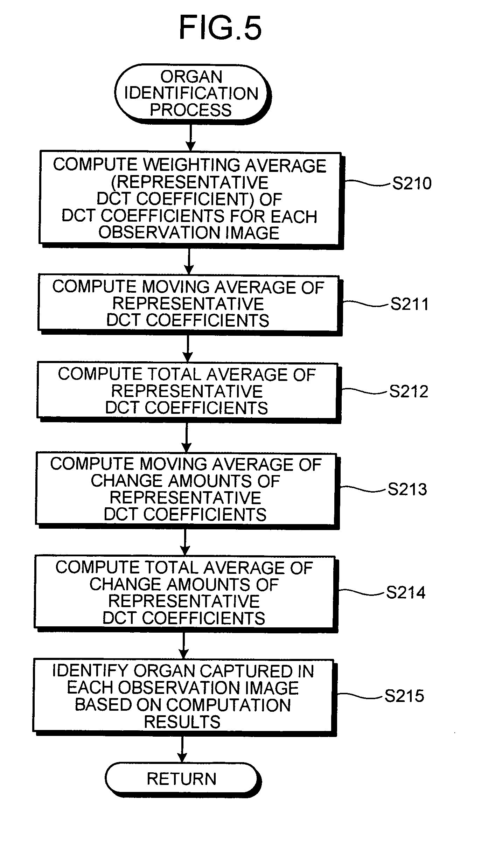

Next, the specific processing procedure of the organ identification process performed by the organ identifying unit 4a will be explained. FIG. 5 is a flowchart illustrating the organ identification processing procedure. As illustrated in FIG. 5, the organ identifying unit 4a first computes a representative DCT coefficient that functions as a weighting average of DCT coefficients for each observation image (Step S210). Then, on the basis of representative DCT coefficients of the series of observation images, the organ identifying unit 4a computes a moving average of the representative DCT coefficients (Step S211), and computes a total average of the representative DCT coefficients (Step S212). Furthermore, the organ identifying unit 4a computes change amounts of the representative DCT coefficients between consecutive observation images of the series of observation images. Then, the organ identifying unit 4a computes a moving average of the change amounts of the representative DCT coefficients (Step S213) and also computes a total average of the change amounts of the representative DCT coefficients (Step S214). After that, the organ identifying unit 4a identifies an organ captured on each observation image (Step S215) on the basis of the computation results performed by Step S211 to S213. Then, the organ identifying unit 4a terminates the organ identification process and returns the system control to Step S102.

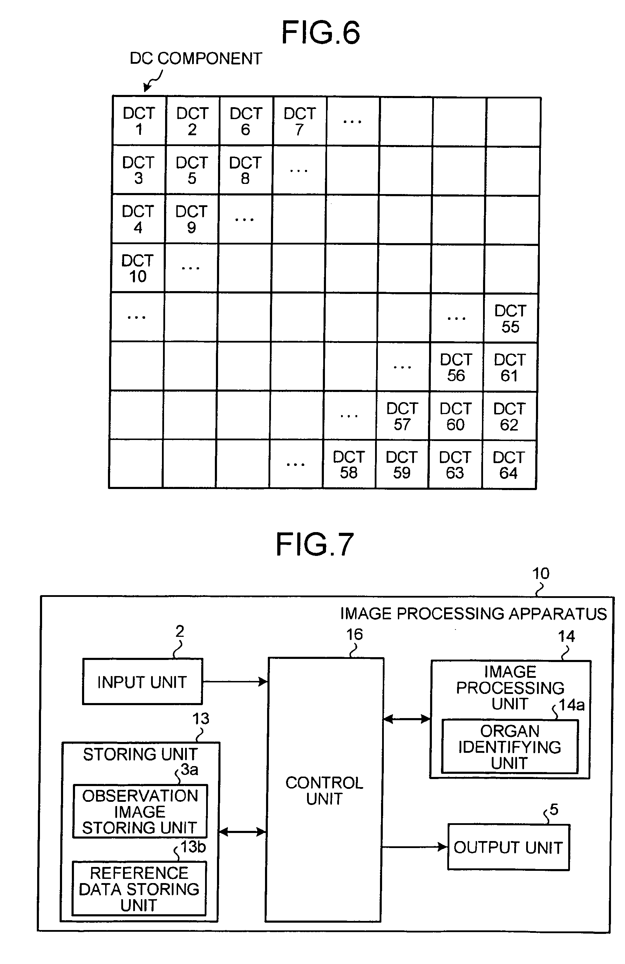

At Step S210, the organ identifying unit 4a first computes a block average of a predetermined number of DCT coefficients from a low frequency component to a high frequency component, for each of $8 \times 8$ pixel blocks. Each block is a process unit at the time of decompression of compressed image data for each observation image.
Specifically, based on $8 \times 8$ pixel blocks illustrated in FIG. 6, the organ identifying unit 4a computes, as the block average, a weighted average of frequency weighted DCT coefficients of "DCT2" to "DCT64", i.e., DCT coefficients "DCT1" to "DCT64" with exception of "DCT1" corresponding to a DC component, or a weighted average of frequency weighted DCT coefficients of one or more DCT coefficients that are previously selected from "DCT2" to "DCT64". In weighting the respective frequencies, it is preferable that high frequencies be weighted more heavily than low frequencies. Furthermore, the organ identifying unit 4a computes, as a representative DCT coefficient, a total average obtained by further averaging the block averages of all of $8 \times 8$ pixel blocks for each observation image.

At Step S211, the organ identifying unit 4a computes, for an image to be processed among the series of observation images, a DCT coefficient average that is an average of representative DCT coefficients of a plurality of observation images that include the image to be processed and are adjacent to the image to be processed in time series. Then, the organ identifying unit 4a associates the computed DCT coefficient average with the image to be processed. In the second embodiment, the organ identifying unit 4a computes the DCT coefficient average by using, for example, 100 observation images adjacent to the image to be processed in time series among the series of observation images. However, the number of observation images for computing the DCT coefficient average can be set to a suitable number in accordance with an imaging interval for capturing the series of observation images. The organ identifying unit 4a sequentially selects an image to be processed among the series of observation images and computes a DCT coefficient average for each selected image to be processed. In this way, the organ identifying unit 4a can obtain a moving average of the representative DCT coefficients over the series of observation images.

At Step S213, the organ identifying unit 4a computes, for an image to be processed among the series of observation images, a DCT-change-amount average that is an average of change amounts of representative DCT coefficients between observation images of the plurality of observation images that include the image to be processed and are adjacent to the image to be processed in time series. Then, the organ identifying unit 4a associates the computed DCT-change-amount average with the image to be processed. In the second embodiment, the organ identifying unit 4a computes the DCT-change-amount average by using, for example, 100 observation images adjacent to the image to be processed in time series among the series of observation images. However, the number of observation images for computing the DCT-change-amount average can be set to a suitable number in accordance with an imaging interval for capturing the series of observation images. The organ identifying unit 4a sequentially selects an image to be processed among the series of observation images and computes the DCT-change-amount average for each selected image to be processed. In this way, the organ identifying unit 4a can obtain a moving average of change amounts of representative DCT coefficients over the series of observation images.

At Step S215, the organ identifying unit 4a first identifies, for an image to be processed among the series of observation images, whether an organ captured on the image to be processed is either an esophagus or a stomach, or either a small intestine or a large intestine in accordance with a magnitude relation between the DCT coefficient average computed at Step S211 and a predetermined DCT criterion. Specifically, on the basis of a total average $F_{dctAve}$ computed at Step S212 and a variable K set previously, the organ identifying unit 4a computes a threshold value $T_{dct}$ that functions as the DCT criterion by using the following Equation (6) and determines whether the DCT coefficient average $F_{dct}$ satisfies the following Inequality (7) with respect to the threshold value $T_{dct}$.

$$T_{dct} = F_{dctAve} + K \quad \cdots \quad (6)$$

$$F_{dct} < T_{dct} \quad \cdots \quad (7)$$

The organ identifying unit 4a identifies that the organ captured on the image to be processed is an esophagus or a stomach when Inequality (7) is satisfied. The organ identifying unit 4a identifies that the mage is a small intestine or a large intestine when Inequality (7) is not satisfied. Then, the organ identifying unit 4a associates the identification result with the image to be processed. Furthermore, the organ identifying unit 4a sequentially selects an image to be processed among the series of observation images and performs a similar identification for each selected image to be processed. In this way, the organ identifying unit 4a identifies whether the organ captured on each observation image of the series of observation images is either an esophagus or a stomach, or either a small intestine or a large intestine.

When it is clear that the series of observation images have been captured in the order of a stomach, a small intestine, and a large intestine, the organ identifying unit 4a sequentially selects an image to be processed from the leading image of the series of observation images. When an observation image not satisfying Inequality (7) is first found, the organ identifying unit 4a identifies that all observation images after the first found observation image are an image on which a small intestine or a large intestine is captured. In this way, the organ identifying unit 4a can more quickly identify whether an observation image is an image on which either an esophagus or a stomach is captured, or an image on which either a small intestine or a large intestine is captured. In other words, when the imaging sequence is predetermined for plural kinds of observation targets, the organ identifying unit 4a identifies that an observation target is a target having the former imaging sequence, and after the organ identifying unit 4a identifies that an observation target is the next observation target, the organ identifying unit 4a can identify that an observation target to be identified after that is the next observation target or an observation target having the next imaging sequence.

Next, the organ identifying unit 4a identifies whether an organ captured on an image to be processed is a small intestine or a large intestine for the image to be processed among the series of observation images that have been identified to be images of a small intestine or a large intestine, in accordance with a magnitude relation between the DCT-change-amount average computed at Step S213 and a predetermined DCT-change-amount criterion. Specifically, on the basis of a total average $F_{dctDiffAve}$ computed at Step S214 and a variable L set previously, the organ identifying unit 4a computes a threshold value $T_{dctDiff}$ that functions as the DCT-change-amount criterion by using the following Equation (8) and determines whether a DCT-change-amount average $F_{dctDiff}$ satisfies the following Inequality (9) for the threshold value $T_{dctDiff}$.

$$T_{dctDiff} = F_{dctDiffAve} + L \quad \cdots \quad (8)$$

$$F_{dctDiff} < T_{dctDiff} \quad \cdots \quad (9)$$

The organ identifying unit 4a identifies that an organ captured on the image to be processed is a large intestine when Inequality (9) is satisfied and identifies that the organ is a small intestine when Inequality (9) is not satisfied. Then, the organ identifying unit 4a associates the identification result with the image to be processed. Furthermore, the organ identifying unit 4a sequentially selects an image to be processed among the series of observation images that have been identified in first to be captured thereon either a small intestine or a large intestine, and performs a similar identification for each selected image to be processed. In this way, the organ identifying unit 4a identifies whether an organ captured on each observation image is a small intestine or a large intestine. In this manner, the organ identifying unit 4a can identify that an organ captured on each image of the series of observation images corresponds to which organ of an esophagus or a stomach, a small intestine, and a large intestine, and associate an identification result with each observation image.

As indicated by Equation (6), the total average $F_{dctAve}$ of representative DCT coefficients is used to compute the threshold value $T_{dct}$ that functions as the DCT criterion. The reason is to reduce an influence induced by an individual difference because each subject has the individual difference with respect to a special feature of an organ. Similarly, as indicated by Equation (8), the total average $F_{dctDiffAve}$ of change amounts of representative DCT coefficients is used to compute the threshold value $T_{dctDiff}$ that functions as the DCT-change-amount criterion. The reason is to reduce an influence induced by the individual difference. Moreover, the variables K and L are set by being inputted by the observer through the input unit 2. Therefore, the variables can be changed appropriately.

As described above, the image processing apparatus 1 according to the second embodiment includes the organ identifying unit 4a that identifies an organ that is an observation target captured on an image to be processed on the basis of a DCT coefficient that is compressed information based on compressed image data of the image to be processed among the series of observation images. The organ identifying unit 4a sequentially selects an image to be processed among the series of observation images and identifies the type of an organ captured on each selected image to be processed. Therefore, the organ identifying unit 4a can quickly identify an organ captured on each observation image of the series of observation images on which a plurality of internal organs such as an esophagus, a stomach, a small intestine, and a large intestine are sequentially captured. Moreover, the organ identifying unit 4a associates the identification result with each observation image. Therefore, the series of observation images can be identified for each captured organ.

In the organ identification process as described above, the organ identifying unit 4a identifies an organ captured on each observation image in block by using Step S215. However, the organ identifying unit 4a can individually perform the identification of Inequality (7) and the identification of Inequality (9). For example, the organ identifying unit 4a performs the identification of Inequality (7) just after Step S212. In this way, the organ identifying unit 4a can perform Step S213 on only the observation image that has been identified to be a image of a small intestine or a large intestine. Therefore, the organ identification process can be performed more quickly.

Moreover, in the organ identification process as described above, the organ identifying unit 4a sequentially performs the identification of Inequality (7) and the identification of Inequality (9) at Step S215. However, the organ identifying unit 4a can perform the identifications in block. For example, the organ identifying unit 4a can calculate a feature vector ($F_{dct}$, $F_{dctDiff}$) expressed by the DCT coefficient average $F_{dct}$ and the DCT-change-amount average $F_{dctDiff}$ for each image to be processed and identify the type of an organ in accordance with an area to which the feature vector belongs on a feature space. Specifically, the organ identifying unit 4a can identify that either an esophagus or a stomach is captured when the feature vector ($F_{dct}$, $F_{dctDiff}$) is in an area satisfying Inequality (7). The organ identifying unit 4a can identify that a large intestine is captured when the feature vector is in an area that is other than the area satisfying Inequality (7) and satisfies Inequality (9).
The organ identifying unit 4a can identify that a small intestine is captured when the feature vector is in an area other than these areas.

Moreover, in the organ identification process as described above, the organ identifying unit 4a identifies an organ on the basis of a DCT coefficient average and a DCT-change-amount average of the plurality of observation images. However, the averages are not necessarily used. The organ identifying unit 4a can identify an organ, for example, on the basis of an individual representative DCT coefficient and a change amount of the individual representative DCT coefficient. In this way, when comparatively loose identification accuracy is requested, an organ identification process can be performed more quickly.

(Third Embodiment)

Next, an image processing apparatus according to the third embodiment of the present invention will be explained. In the second embodiment as described above, the organ identifying unit 4a identifies an organ captured on each observation image on the basis of representative DCT coefficients of an observation image and a change amount of the representative DCT coefficients. In the third embodiment, the organ identifying unit calculates a feature vector based on a plurality of DCT coefficients for each observation image and identifies an organ on the basis of the feature vector.

FIG. 7 is a block diagram illustrating the main configuration of an image processing apparatus 10 according to the third embodiment. As illustrated in FIG. 7, the image processing apparatus 10 includes a storing unit 13, an image processing unit 14, and a control unit 16 instead of the storing unit 3, the image processing unit 4, and the control unit 6, respectively, based on the configuration of the image processing apparatus 1. The storing unit 13 further includes a reference data storing unit 13b that stores therein reference data for use in an organ identification process to be described below, based on the configuration of the storing unit 3. The image processing unit 14 includes an organ identifying unit 14a instead of the organ identifying unit 4a based on the configuration of the image processing unit 4. Other configurations are identical to those of the image processing apparatus 1.
The same reference numerals are given to the same components.

In the image processing apparatus 10, the organ identifying unit 14a identifies that a captured organ corresponds to which organ of an esophagus or a stomach, a small intestine, and a large intestine, by using the difference of frequency distribution between observation images of captured organs. Specifically, when a series of observation images are stored as compressed image data compressed by a DCT compression encoding mode, the organ identifying unit 14a computes a feature vector based on a plurality of DCT coefficients as an index indicated by the frequency distribution of an observation image and performs an identification on the basis of the computed feature vectors and predetermined reference data that functions as dictionary data stored in the reference data storing unit 13b.

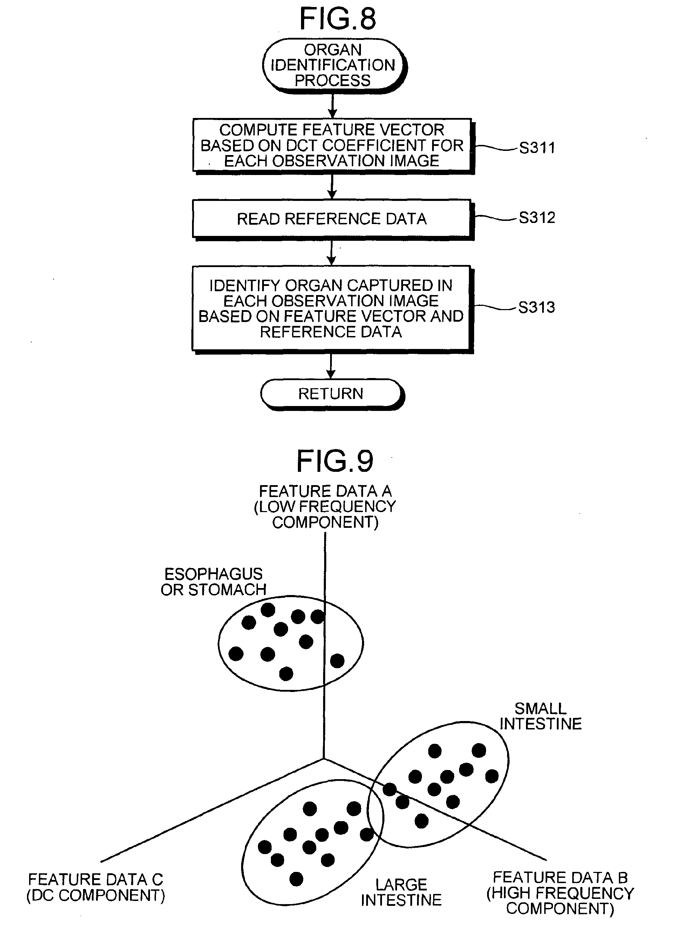

FIG. 8 is a flowchart illustrating the process procedure of an organ identification process performed by the organ identifying unit 14a. The image processing apparatus 10 processes the series of observation images in accordance with the processing procedure illustrated in FIG. 2 similarly to the image processing apparatus 1. The organ identification processing procedure illustrated in FIG. 8 is executed as Step S102 illustrated in FIG. 2. As illustrated in FIG. 8, the organ identifying unit 14a first computes a feature vector on the basis of a DCT coefficient for each observation image (Step S311) and reads reference data from the reference data storing unit 13b (Step S312). Then, the organ identifying unit 14a identifies an organ captured on each observation image on the basis of the computed feature vector and the read reference data (Step S313). After that, the organ identifying unit 14a terminates the organ identification process and returns the system control to Step S102.

At Step S311, the organ identifying unit 14a first computes, with respect to an image to be processed in the series of observation images, a block representative value of low frequency components and a block representative value of high frequency components on the basis of one or more DCT coefficients, for each of 8 x 8 pixel blocks. Each block is a process unit at the time of decompression of compressed image data for each observation image. Specifically, based on "DCT1" to "DCT64 of the 8 x 8 pixel blocks illustrated in FIG. 6, the organ identifying unit 14a computes, for example, a weighted average of frequency weighted DCT coefficients of "DCT2" to "DCT10" as the block representative value of the low frequency components and computes a weighted average of frequency weighted DCT coefficients of "DCT55" to "DCT64" as the block representative value of the high frequency components. In weighting the respective frequencies, it is preferable that high frequencies be weighted more heavily than low frequencies.

Furthermore, the organ identifying unit 14a computes an average of the block representative values of the low frequency components of all of the $8 \times 8$ pixel blocks within the image to be processed, an average of the block representative values of the high frequency components of all of the $8 \times 8$ pixel blocks within the image to be processed, and an average of "DCT1"s indicative of their respective DC components of all of the $8 \times 8$ pixel blocks within the image to be processed, thereby obtaining the three averages as feature data A to C. Then, the organ identifying unit 14a associates a vector on a feature space that is indicated by the feature data A to C with the image to be processed as a feature vector indicative of the frequency distribution of the image to be processed. Furthermore, the organ identifying unit 14a sequentially selects an image to be processed among the series of observation images and performs a similar process for each selected image to be processed. In this way, the organ identifying unit 14a computes the feature vector of each observation image.

At Step S312, the organ identifying unit 14a reads, for example, reference data that functions as a class dictionary in which the respective organs are previously classified on a feature space as illustrated in FIG. 9. Then, at Step S313, the organ identifying unit 14a identifies the type of an organ to which the feature vector of each observation image computed at Step S311 belongs, on the basis of the reference data read at Step S312, for example, by using a well-known identification method such as a kNN method (k-Nearest Neighbor Method) or a subspace method. At that time, the organ identifying unit 14a sequentially selects an image to be processed among the series of observation images and identifies the type of an organ to which the feature vector belongs for each selected image to be processed. In this way, the organ identifying unit 14a identifies that the organ captured on each observation image corresponds to which organ of an esophagus or a stomach, a small intestine, and a large intestine, and associates an identification result with each observation image.

As described above, in the image processing apparatus 10 according to the third embodiment, the organ identifying unit 14a computes a feature vector based on DCT coefficients for each observation image and identifies the organ captured on each observation image on the basis of the computed feature vector and predetermined reference data. Therefore, the organ identifying unit 14a can quickly identify the organ captured on each observation image of the series of observation images on which the plurality of internal organs such as an esophagus, a stomach, a small intestine, and a large intestine are sequentially captured. Moreover, the organ identifying unit 14a associates the identification result with each observation image. Therefore, the series of observation images can be identified for each captured organ.

In the organ identification process as described above, the organ identifying unit 14a computes the feature vector on the basis of three pieces of feature data A to C and identifies the type of an organ. However, the number of pieces of feature data is not limited to three. The organ identifying unit 14a can compute a feature vector on the basis of two pieces of feature data or four or more pieces of feature data. For example, at Step S311, the organ identifying unit 14a sets each of DCT coefficients "DCT1" to "DCT64" of each of the 8 × 8 pixel blocks to a block representative value, and computes, as feature data, an average value of the block representative values for each corresponding block in all of the 8 × 8 pixel blocks within the image to be processed. In this way, the organ identifying unit 14a can obtain a feature vector consisting of 64-dimensional feature data maximally. In this manner, the organ identifying unit 14a can perform an organ identification based on a feature vector on which all frequency components of DCT coefficients are reflected. Therefore, the organ identifying unit 14a can perform the organ identification with higher accuracy. However, because a processing time for deriving the feature vector increases due to the increase of the number of dimensions, it is preferable that the number of dimensions be appropriately set in accordance with desired identification accuracy.

As above, the best modes of realizing the present invention have been explained as the first to third embodiments. However, the present invention is not limited to the first to third embodiments as described above. The present invention can be variously modified within a range that does not deviate from an object of the present invention.

For example, in the organ identification process according to the first to third embodiments as described above, the organ identifying units 4a and 14a perform an organ identification on all observation images of the series of observation images. However, the organ identifying units 4a and 14a can perform an organ identification, for example, on only observation images corresponding to a predetermined number of images or up to a predetermined image number. Alternatively, the organ identifying units 4a and 14a can designate a desired organ and perform an organ identification on up to observation images on which the designated organ is captured. In this way, the organ identifying units 4a and 14a can more quickly perform an organ identification process on only observation images on which a desired organ is captured.

In the first to third embodiments as described above, the series of observation images that are processed by the image processing apparatus 1 or 10 are sequentially captured in the order of an esophagus, a stomach, a small intestine, and a large intestine. However, the present invention can be applied even if two or more organs are sequentially captured among an esophagus or a stomach, a small intestine, and a large intestine.

INDUSTRIAL APPLICABILITY

As described above, an image processing apparatus and an image processing program according to the present invention are useful in processing a series of observation images on which a plurality of observation targets are sequentially captured.

**Claims**

1. An image processing apparatus that processes a series of observation images on which a plurality of observation targets are sequentially captured, the image processing apparatus comprising
   a target identifying unit that identifies, at least on the basis of compressed information based on compressed image data of an image to be processed among the series of observation images, an observation target captured on the image to be processed.

2. The image processing apparatus according to claim 1, wherein the target identifying unit identifies the observation target captured on the image to be processed in accordance with a magnitude relation between the compressed information of the image to be processed and a predetermined information-amount criterion.

3. The image processing apparatus according to claim 1, wherein the target identifying unit computes an information amount average of the compressed information of the plurality of observation images that include the image to be processed and that are adjacent to the image to be processed in time series within a predetermined range, and identifies the observation target captured on the image to be processed in accordance with a magnitude relation between the information amount average and a predetermined information-amount criterion.

4. The image processing apparatus according to claim 2 or 3, wherein the predetermined information-amount criterion is determined on the basis of a total average of the compressed information of the series of observation images.

5. The image processing apparatus according to claim 1, wherein the target identifying unit computes a change amount of the compressed information between the image to be processed and the observation image adjacent to the image to be processed in time series within a predetermined range, and identifies the observation target captured on the image to be processed in accordance with a magnitude relation between the change amount and a predetermined change-amount criterion.

6. The image processing apparatus according to claim 1, wherein the target identifying unit computes a change-amount average of change amounts of the compressed information between the respective observation images of the plurality of observation images that include the image to be processed and that are adjacent to the image to be processed in time series within a predetermined range, and identifies the observation target captured on the image to be processed in accordance with a magnitude relation between the change-amount average and a predetermined change-amount criterion.

7. The image processing apparatus according to claim 5 or 6, wherein the predetermined change-amount criterion is determined on the basis of a total average of change amounts of the compressed information between the respective observation images of the series of observation images.

8. The image processing apparatus according to claim 1, wherein the target identifying unit computes a feature vector on the basis of the compressed information of the image to be processed, and identifies the observation target captured on the image to be processed on the basis of the feature vector and predetermined reference data.

9. The image processing apparatus according to claim 1, wherein the compressed information is a file size of the compressed image data, a plurality of DCT coefficients that are computed at the time of decompression of the compressed image data, or a statistic of the plurality of DCT coefficients.

10. The image processing apparatus according to claim 1, wherein the target identifying unit sequentially selects an image to be processed among the series of observation images, and identifies an observation target for each selected image to be processed.

11. The image processing apparatus according to claim 1, wherein
the series of observation images are an image group on which two or more organs among an esophagus or a stomach, a small intestine, and a large intestine are sequentially captured, and
the target identifying unit identifies that the observation target captured on the image to be processed corresponds to which organ of the two or more organs.

12. An image processing program causing an image processing apparatus that processes a series of observation images on which a plurality of observation targets are sequentially captured, to process the series of observation images, the image processing program causing the image processing apparatus to perform:

identifying, at least on the basis of compressed information based on compressed image data of an image to be processed among the series of observation images, an observation target captured on the image to be processed.

# FIG.1

1

IMAGE PROCESSING APPARATUS

2
INPUT UNIT

3
STORING UNIT
3a
OBSERVATION IMAGE STORING UNIT

6
CONTROL UNIT

4
IMAGE PROCESSING UNIT
4a
ORGAN IDENTIFYING UNIT

5
OUTPUT UNIT

# FIG.2

START

READ OBSERVATION IMAGES —— S101

ORGAN IDENTIFICATION PROCESS —— S102

OUTPUT IDENTIFICATION RESULT —— S103

END

# FIG.3

```
        ┌─────────────────────┐
        │        ORGAN        │
        │   IDENTIFICATION    │
        │      PROCESS        │
        └─────────────────────┘
                  │
                  ▼
    ┌──────────────────────────────┐
    │  COMPUTE MOVING AVERAGE OF    │──── S111
    │         FILE SIZES            │
    └──────────────────────────────┘
                  │
                  ▼
    ┌──────────────────────────────┐
    │  COMPUTE TOTAL AVERAGE OF     │──── S112
    │         FILE SIZES            │
    └──────────────────────────────┘
                  │
                  ▼
    ┌──────────────────────────────┐
    │  COMPUTE MOVING AVERAGE OF    │──── S113
    │ CHANGE AMOUNTS OF FILE SIZES  │
    └──────────────────────────────┘
                  │
                  ▼
    ┌──────────────────────────────┐
    │  COMPUTE TOTAL AVERAGE OF     │──── S114
    │ CHANGE AMOUNTS OF FILE SIZES  │
    └──────────────────────────────┘
                  │
                  ▼
    ┌──────────────────────────────┐
    │   IDENTIFY ORGAN CAPTURED IN  │
    │   EACH OBSERVATION IMAGE      │──── S115
    │   BASED ON COMPUTATION        │
    │         RESULTS               │
    └──────────────────────────────┘
                  │
                  ▼
        ┌─────────────────────┐
        │       RETURN        │
        └─────────────────────┘
```

# FIG.4-1

IMAGE NUMBER OF OBSERVATION IMAGES

# FIG.4-2

IMAGE NUMBER OF OBSERVATION IMAGES

# FIG.4-3

# FIG.5

```
ORGAN
IDENTIFICATION
PROCESS
```

COMPUTE WEIGHTING AVERAGE
(REPRESENTATIVE
DCT COEFFICIENT) OF
DCT COEFFICIENTS FOR EACH
OBSERVATION IMAGE — S210

COMPUTE MOVING AVERAGE OF
REPRESENTATIVE
DCT COEFFICIENTS — S211

COMPUTE TOTAL AVERAGE OF
REPRESENTATIVE
DCT COEFFICIENTS — S212

COMPUTE MOVING AVERAGE OF
CHANGE AMOUNTS OF
REPRESENTATIVE
DCT COEFFICIENTS — S213

COMPUTE TOTAL AVERAGE OF
CHANGE AMOUNTS OF
REPRESENTATIVE
DCT COEFFICIENTS — S214

IDENTIFY ORGAN CAPTURED IN
EACH OBSERVATION IMAGE
BASED ON COMPUTATION
RESULTS — S215

```
RETURN
```

# FIG.6

DC COMPONENT

| DCT 1 | DCT 2 | DCT 6 | DCT 7 | . . . | | | |
|---|---|---|---|---|---|---|---|
| DCT 3 | DCT 5 | DCT 8 | . . . | | | | |
| DCT 4 | DCT 9 | . . . | | | | | |
| DCT 10 | . . . | | | | | | |
| . . . | | | | | | . . . | DCT 55 |
| | | | | | | . . . | DCT 56 | DCT 61 |
| | | | | | . . . | DCT 57 | DCT 60 | DCT 62 |
| | | | . . . | DCT 58 | DCT 59 | DCT 63 | DCT 64 |

# FIG.7

# FIG.8

ORGAN
IDENTIFICATION
PROCESS

COMPUTE FEATURE VECTOR
BASED ON DCT COEFFICIENT FOR
EACH OBSERVATION IMAGE — S311

READ REFERENCE DATA — S312

IDENTIFY ORGAN CAPTURED IN
EACH OBSERVATION IMAGE
BASED ON FEATURE VECTOR AND
REFERENCE DATA — S313

RETURN

# FIG.9

FEATURE DATA A
(LOW FREQUENCY
COMPONENT)

ESOPHAGUS
OR STOMACH

SMALL
INTESTINE

FEATURE DATA C
(DC COMPONENT)

LARGE
INTESTINE

FEATURE DATA B
(HIGH FREQUENCY
COMPONENT)

## INTERNATIONAL SEARCH REPORT

| | International application No. |
| --- | --- |
| | PCT/JP2008/057154 |

A. CLASSIFICATION OF SUBJECT MATTER
*A61B1/00*(2006.01)i, *A61B1/04*(2006.01)i, *G06T1/00*(2006.01)i

According to International Patent Classification (IPC) or to both national classification and IPC

B. FIELDS SEARCHED

Minimum documentation searched (classification system followed by classification symbols)
A61B1/00, A61B1/04, G06T1/00

Documentation searched other than minimum documentation to the extent that such documents are included in the fields searched
Jitsuyo Shinan Koho            1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br><br>A | JP 2004-154176 A  (Olympus Corp.),<br>03 June, 2004 (03.06.04),<br>Par. Nos. [0033] to [0041]<br>& US 2007/0161858 A1 | 1,2,5,9,10,<br>12<br>3,4,6-8,11 |
| A | JP 2004-321605 A  (Olympus Corp.),<br>18 November, 2004 (18.11.04),<br>Par. Nos. [0067], [0068]<br>& US 2004/0215059 A1    & WO 2004/096030 A1 | 1-12 |
| A | JP 2004-337596 A  (Olympus Corp.),<br>02 December, 2004 (02.12.04),<br>Par. Nos. [0138] to [0149]<br>& US 2004/0225223 A1    & EP 1618828 A1<br>& EP 1857042 A2       & WO 2004/096025 A1<br>& CA 2523302 A          & KR 10-2006-0003050 A<br>& CN 1777390 A | 1-12 |

☐ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    16 June, 2008 (16.06.08) | Date of mailing of the international search report<br>    01 July, 2008 (01.07.08) |
| --- | --- |
| Name and mailing address of the ISA/<br>    Japanese Patent Office | Authorized officer |
| Facsimile No. | Telephone No. |

Form PCT/ISA/210 (second sheet) (April 2007)

**REFERENCES CITED IN THE DESCRIPTION**

**Patent documents cited in the description**

- JP 2006320585 A